# FASCICULE DE BREVET EUROPEEN

(11) **EP 1 494 593 B1**
(45) Date de publication et mention de la délivrance du brevet: **20.07.2005**
(21) Numéro de dépôt: 03735774.6
(22) Date de dépôt: 07.03.2003
(51) Int. Cl.: A61B 17/068

(54) **APPAREIL DE STOCKAGE, DE DISTRIBUTION ET DE POSE D'ATTACHES CHIRURGICALES EN "1" COUCHE**
VORRICHTUNG ZUR AUFBEWAHRUNG, VERTEILUNG UND PLATZIERUNG VON I-FÖRMIGEN CHIRURGISCHEN AUFSÄTZEN
DEVICE FOR STORING, DISTRIBUTING AND PLACING I -SHAPED SURGICAL ATTACHMENTS

(30) Priorité: 08.03.2002 FR 0203082
(43) Date de publication de la demande: 12.01.2005
(73) Titulaire: SOFRADIM PRODUCTION, 01600 Trévoux (FR)
(72) Inventeur: BAILLY, Pierre, F-69300 Caluire (FR); EVRARD, Frédéric, F-69400 Limas (FR); THERIN, Michel, F-69004 Lyon (FR); BENCHETRIT, Salomon, F-69300 Caluire (FR)
(74) Mandataire: Brédeville, Odile Marie
(86) Numéro de dépôt international: PCT/FR2003/000749
(87) Numéro de publication internationale: WO 2003/075773

(56) Documents cités:
- WO-A-00/67644
- WO-A-99/39645
- US-A- 4 448 194

## Description

L'invention concerne un appareil de distribution et de pose d'attaches chirurgicales en "I" pouvant, par exemple, être utilisées pour fixer des renforts pariétaux et viscéraux.

Elle vise plus spécialement à remplacer les attaches et agrafes métalliques permanentes par des attaches en matière plastique, par exemple, en matière résorbable, ayant une fonction temporaire, en rapport avec le temps de la réhabilitation tissulaire de renfort, tout en procurant un ancrage excluant les risques de pincement nerveux.

On connaît déjà dans l'industrie du textile une attache en matière plastique ayant la forme générale d'un "I", c'est-à-dire composée d'une tête, d'un filament de liaison, et d'un pied. Cette attache est distribuée et mise en place au moyen d'un appareil en forme de pistolet dont le canon est prolongé par une aiguille creuse et dont le corps est muni d'une commande manuelle d'actionnement. Pour relier divers éléments en nappe, par exemple une étiquette sur un vêtement, l'aiguille est engagée transversalement dans ces éléments, par enfoncement manuel, puis l'actionnement de la commande manuelle provoque, par le déplacement d'un poussoir disposé dans l'axe longitudinal du canal de l'aiguille:
- le prélèvement d'une attache faisant partie d'une barrette d'attaches, en attente dans un magasin,
- le déplacement de la tête de l'attache dans le canal axial de l'aiguille creuse,
- la traversée des nappes par la tête de l'attache circulant dans l'aiguille,
- puis, dès que le filament de liaison vient au contact des nappes, son rabattement contre la tête et son engagement dans les nappes avec le reste de la tête, jusqu'à ce que la tête soit complètement disposée de l'autre côté des nappes à assembler.

En fin d'actionnement, un moyen, et par exemple un ressort, rappelle le poussoir et l'aiguille est retirée manuellement. Sous l'action de l'élasticité de la matière constituant l'attache, la tête de celle-ci reprend sa position initiale et revient sensiblement perpendiculairement au filament de liaison, en assurant l'ancrage de l'attache sur la face arrière des nappes. Celles-ci sont alors emprisonnées entre la tête et le pied de l'attache qui s'étend lui aussi transversalement, mais sur la face avant des nappes.

Il a été envisagé de transposer ce type d'attache pour assurer des ligatures chirurgicales, par exemple d'un renfort textile contre des tissus biologiques, en réalisant l'attache en matériau plastique et, notamment, en matériau biorésorbable, et en la distribuant au moyen d'un distributeur équivalent. Il s'est alors révélé des inconvénients provenant de l'encombrement généré par la barrette d'attaches saillant du nez du distributeur, et de ceux inhérents au respect des spécifications chirurgicales et, en particulier, de la stérilisation. Il en est ainsi pour les appareils décrits dans US-A-4 448 194 ET WO98/51179.

Pour remédier à ces inconvénients, le document WO00/67644 décrit un appareil de distribution et de pose comprenant un corps de poignée, équipé de premiers et seconds moyens de commande et d'un embout, et un élément longiforme, rapporté et fixé sur l'embout, appareil dans lequel l'élément longiforme est composé d'un corps de forme générale tubulaire contenant :
- à proximité de son extrémité libre, un coulisseau-magasin contenant lui-même des attaches en "I" couché ayant chacune une barre d'ancrage, une barre d'arrêt, et une barrette de liaison, ledit coulisseau étant, d'une part, déplaçable dans l'élément longiforme par une tige longitudinale actionnable par les premiers moyens de commande de la poignée, et, d'autre part, solidaire d'une aiguille creuse fendue et longitudinale, saillant de son extrémité libre et pouvant saillir de cet élément longiforme,
- et, dans son corps tubulaire, d'une part, des moyens de distribution une à une des attaches stockées, cette distribution comprenant le transfert de la barre d'ancrage de la première attache dans un logement d'accueil disposé dans le prolongement de l'aiguille creuse, et, d'autre part, un poussoir d'éjection déplaçable par les seconds moyens de commande de la poignée et disposé dans l'alignement de l'aiguille creuse pour pousser dans celle-ci la barre d'ancrage de la première attache en attente.

Dans cet appareil, les attaches sont soumises, dans leur magasin de stockage, à l'effort permanent d'une lame de poussée qui les sollicite en direction de l'extrémité libre de l'appareil et qui tend, en combinaison avec une rampe pentue, à chasser la barre d'ancrage de la première attache dans un logement d'accueil ménagé derrière l'aiguille fendue, mais obturé au repos par le poussoir. L'actionnement des moyens de commande de la poignée provoque, dans une première phase, le déplacement du coulisseau afin que l'aiguille soit en saillie de l'extrémité libre de l'élément longiforme, ce déplacement s'effectuant sans déplacement du poussoir afin de former le logement d'accueil qui reçoit alors la barre d'ancrage de la première attache.

Dans une deuxième phase, le poussoir est déplacé par les seconds moyens de commande pour chasser la barre d'ancrage de l'attache dans l'aiguille, puis de l'aiguille dans les tissus biologiques devant la recevoir. En fin de distribution, la barre d'arrêt de l'attache en cours de pose passe contre une rampe inclinée du coulisseau qui est censée la faire pivoter pour qu'elle ne vienne pas en butée sur les tissus, mais au contraire se couche contre eux.

Il se révèle en pratique que cette rampe est insuffisante pour faire pivoter la barre d'arrêt de manière satisfaisante, de sorte qu'elle peut encore buter contre le renfort, en gênant la distribution et le mouvement de redressement que doit effectuer la barre d'ancrage dans les tissus biologiques.

Il faut ici remarquer que, à la différence des attaches utilisées pour lier des pièces textiles, dans lesquelles la barre d'ancrage se redresse derrière les nappes, mais dans l'atmosphère, pour prendre par l'élasticité de son matériau constitutif une position parallèle aux nappes, la barre d'ancrage d'une attache chirurgicale doit se redresser dans les tissus biologiques, c'est-à-dire en s'opposant à leur résistance. Ce redressement nécessite un effort supérieur aux capacités élastiques du matériau et est mal pris en compte dans les appareils de l'état de la technique, de sorte que le positionnement de la barre d'ancrage et l'ancrage de l'attache dans les tissus biologiques sont irréguliers.

Ces inconvénients sont aggravés avec les attaches en matériau ayant une faible élasticité ou nervosité, comme c'est le cas pour les matériaux biorésorbables, puisque lors de la distribution de l'attache dans les tissus, les propriétés mécaniques du matériau réduisent les capacités de redressement élastique de ses barres, respectivement d'ancrage et d'arrêt, ce qui conduit à un mauvais ancrage dans les tissus.

Enfin, avec des attaches en matériau biorésorbables ou à faible élasticité, la pression permanente sur elles des moyens à ressort les poussant vers la sortie du magasin tend à les déformer par fluage. Cela génère des accidents de distribution entraînant la mise au rebut de l'appareil.

La présente invention a pour objet de foumir un appareil de stockage, de distribution, et de pose d'attaches chirurgicales qui réduise les contraintes exercées sur les attaches en attente dans le magasin et qui facilite leur pose par une organisation de leurs mouvements de distribution.

Cet appareil reprend les éléments essentiels de celui décrit dans le document WO00/67644 décrit ci-dessus, mais les aménage.

A cet effet, le logement d'accueil du coulisseau-magasin contient, au repos, la barre d'ancrage de la première attache en attente de pose, attache dont la barre d'arrêt disposée à l'extrémité du magasin est libre de tout effort de poussée, tandis que ce coulisseau-magasin comporte :
- au-dessous de l'aiguille et dans la trajectoire de sortie du magasin de ladite barre d'arrêt, un doigt transversal apte, lors de la distribution de l'attache par déplacement de sa barre d'ancrage au moyen du poussoir, à recevoir l'appui de l'extrémité aval de cette barre d'arrêt pour la forcer à basculer en soulevant son extrémité amont,
- à côté de l'aiguille, une nervure saillant vers le bas et apte à guider le basculement et le déplacement de l'extrémité amont de la barre d'arrêt, pour que cette dernière ne vienne pas buter contre l'aiguille,
- et, sur le doigt transversal, une face pentue chassant, transversalement et hors de ce doigt, l'extrémité aval de la barre d'arrêt.

Ainsi, lorsque l'appareil est au repos, la première attache et les suivantes ne sont soumises à aucun effort de poussée, y compris la première attache dont la barre d'ancrage est déjà mise en place dans le logement d'accueil du coulisseau-magasin. Grâce à cela, les attaches stockées sont conservées dans leur état d'origine et ne risquent donc pas de fluer, même avec des matériaux faiblement élastiques.

Lors de la distribution de l'attache, le doigt, la nervure et les pentes du coulisseau, qui coopèrent successivement avec la barre d'arrêt, permettent, dans une première phase, d'utiliser le mouvement de déplacement communiqué par le poussoir à la barre d'ancrage pour faire basculer la barre d'arrêt et pour l'orienter sensiblement parallèlement au renfort, et, dans une deuxième phase, d'utiliser la barre d'arrêt pour retenir la barre d'ancrage et la forcer à basculer dans les tissus biologiques pour prendre une position parallèle à ce renfort textile.

Ainsi, en fin de pose et sans que des contraintes excessives aient été exercées sur l'attache, celle-ci est toujours parfaitement positionnée et assure donc un parfait ancrage.

Dans une forme d'exécution de l'invention, la barre d'arrêt de la première attache est, dans le magasin, au contact des barres d'arrêt des autres attaches stockées, et la barre d'arrêt de la dernière attache est au contact, mais sans effort significatif, avec l'extrémité d'une tige de butée, déplaçable pas à pas, cette tige de butée étant reliée à des moyens aptes, en fin de mouvement de distribution de la première attache dans les tissus et par le mouvement des seconds moyens de commande de la poignée, à la déplacer de la longueur d'une barre d'arrêt d'une attache, afin que, lors du retour du coulisseau-magasin dans sa position de départ et de repos dans le corps tubulaire, le contact entre l'extrémité de cette tige de butée et la barre d'arrêt de la dernière attache provoque le déplacement de la rangée d'attaches dans le magasin, et le transfert de la barre d'ancrage de la première attache dans le logement d'accueil.

Ainsi, après distribution de la première attache, le rechargement du logement d'accueil s'effectue automatiquement, lors du retour du coulisseau dans le corps, par butée de la barre d'arrêt de la dernière attache contre la tige de butée, qui ne s'est avancée que d'un pas correspondant à la longueur de la barre d'arrêt d'une attache.

Avantageusement, le coulisseau-magasin est formé par deux coquilles en matière plastique s'assemblant selon leur plan médian, longitudinal et vertical, la coquille solidaire de l'aiguille creuse fendue comportant deux rainures longitudinales, disposées près de ses bords et aptes à recevoir, l'une, les barres d'arrêt des attaches, l'autre, les barres d'ancrage, la rainure pour les barres d'arrêt débouchant librement de l'extrémité avant du coulisseau alors que la rainure pour les barres d'ancrage se prolonge par un plan incliné de guidage en direction de l'aiguille, tandis que l'autre coquille comporte un canal, longitudinal et coaxial à l'aiguille, pour le poussoir d'éjection, et, dans le logement d'accueil de l'aiguille, un plan de repos incliné verticalement pour la barrette de liaison d'une attache.

D'autres caractéristiques et avantages ressortiront de la description qui suit en référence au dessin schématique annexé représentant une forme d'exécution de l'appareil selon l'invention.
Figure 1 est une vue de côté en élévation d'une attache,
Figure 2 est une vue générale de l'appareil, en partie élévation, et en partie en coupe au niveau de sa poignée et des moyens de liaison de cette poignée avec le corps longiforme,
Figure 3 est une vue partielle en coupe longitudinale de l'élément longiforme représenté, à échelle agrandie, par rapport à la figure 2,
Figure 4 est une vue en perspective éclatée à échelle agrandie, de l'extrémité antérieure de l'élément longiforme et du coulisseau-magasin,
Figure 5 est une vue de face en élévation et à échelle agrandie de l'extrémité antérieure de l'élément longiforme,
Figure 6 est une vue partielle en coupe suivant VI-VI de figure 5,
Figure 7 est une vue partielle en coupe, à échelle agrandie, suivant VII-VII de figure 3, lorsque l'appareil est au repos,
Figures 8 et 9 sont des vues en coupe similaires à la figure 7, montrant l'arbre à gorges dans les phases, respectivement, d'avancement d'un pas de la douille d'armement et de l'arbre à gorges et de retour en position de repos de cette douille,
Figure 10 est une vue partielle en coupe transversale de la poignée, lorsqu'elle est en cours d'actionnement,
Figures 11 à 16 sont des vues en coupe longitudinale de l'élément longiforme et de ses composants, lorsqu'ils sont, respectivement, au repos, en phase de sortie de l'aiguille, en phase de début et de fin d'éjection, en phase de retrait de l'éjecteur, et en phase de retrait du coulisseau.

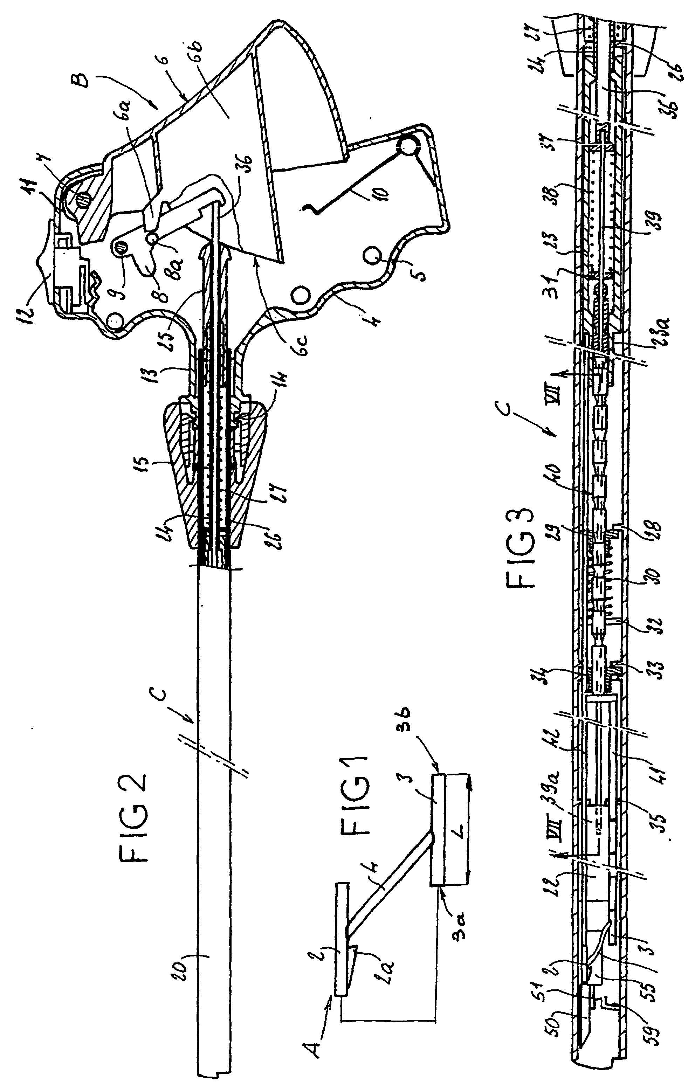

L'appareil est destiné à distribuer des agrafes A ayant, comme montré à la figure 1, la forme générale d'un "I" couché et composé de deux barres parallèles, respectivement, d'ancrage 2 et d'arrêt 3, reliées par une barrette de liaison inclinée 4. La barre d'ancrage 2 comporte un cran 2a saillant de sa partie qui est engagée en premier dans un tissu humain. Cette attache est réalisée en matière plastique et notamment en matériau biocompatible, de préférence biorésorbable, mais pouvant aussi ne pas être biorésorbable.

L'appareil en lui-même est composé de deux éléments, à savoir un ensemble de préhension B et un élément longiforme C.

L'élément de préhension B comprend un corps de poignée 4, réalisé en deux parties symétriques assemblées par soudage à ultrasons ou par des rivets ou vis 5, une poignée 6 articulée sur un axe transversal 7 du corps, un levier de commande 8 dont l'extrémité supérieure est articulée sur un axe transversal 9 du corps, un ressort de torsion 11 disposé autour de l'axe 7 et rappelant la poignée 6 à l'extérieur du corps, un verrou 12 monté coulissant dans le corps et pouvant caler la poignée en position de repos, et un embout 13 faisant partie du corps et sur lequel est calée, avec des moyens d'encliquetage 14 actionnables par une douille pivotante 15, l'extrémité postérieure de l'élément longiforme C. Un ressort en épingle 10 venant en contact avec la poignée 6, sensiblement à mi course de celle-ci, forme un seuil sensitif qui prévient l'opérateur que l'aiguille est totalement sortie, sans éjection d'attache. Pour terminer le cycle de pose jusqu'à l'éjection d'une attache, il faut exercer sur la poignée 6 un effort plus important. Tous les éléments de la poignée, à l'exception des axes, des vis et des ressorts, sont obtenus par moulage de matière plastique.

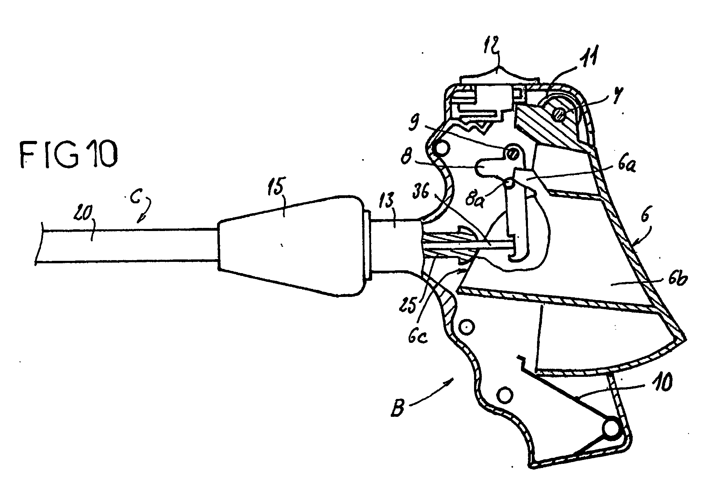

La poignée 6 comporte une fourchette à deux doigts 6a, aptes à coopérer avec des tenons 8a, saillant latéralement du levier 8, pour faire pivoter ce levier vers l'avant. Les doigts 6a, les tenons 8a, et le levier 8 constituent les premiers moyens de commande de l'appareil. Les seconds moyens de commande sont constitués par deux joues 6b portant, à leur extrémité antérieure, un profil en came 6c, visible figures 2 et 10.

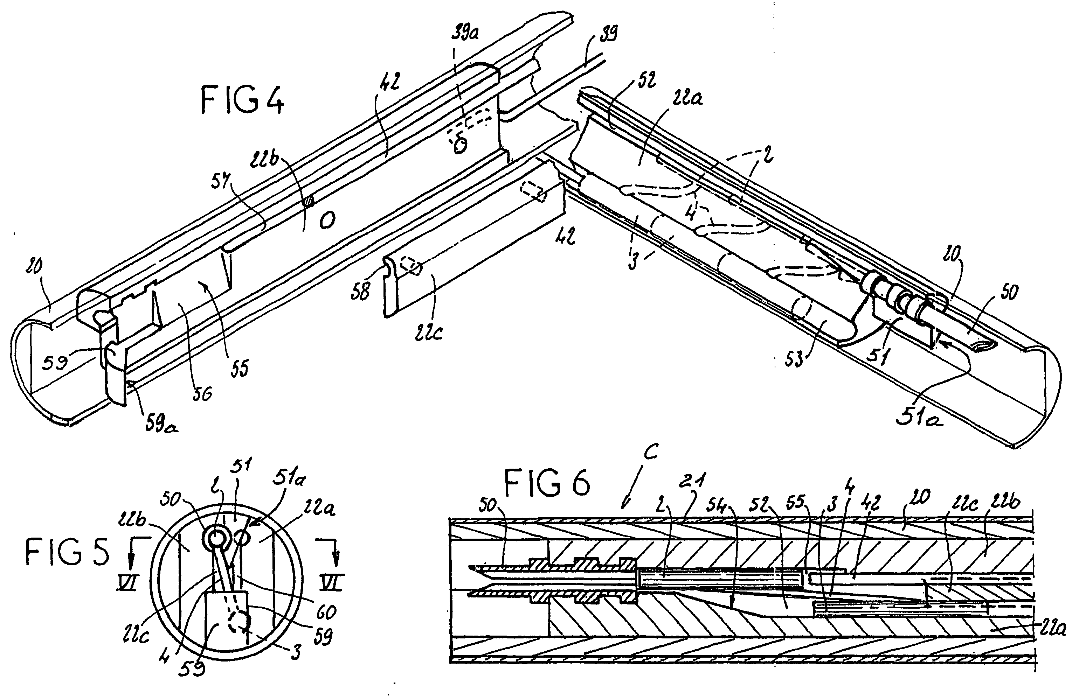

Comme montré figure 2 et 3, l'élément longiforme C comprend un corps tubulaire 20 dans lequel sont montés coulissants un coulisseau-magasin 22 et une cage 23. Cette dernière est reliée, par un tronçon tubulaire métallique 24, à un piston 25 pénétrant dans le corps de poignée et venant en appui sur les profils en came 6c. Le corps tubulaire 20 est lui-même composé de deux coquilles semi cylindriques en matière plastique assemblées dans leur plan diamétral, par soudure ou collage, chacune des coquilles étant elle-même monolithique ou composée de deux éléments assemblés par emboîtage et rivetage de leurs extrémités communes. Ce mode de liaison étant à la portée de l'homme de l'art, il n'a pas été jugé utile de le représenter pour ne pas compliquer les dessins. De préférence et comme montré figure 6, le corps 20 en plusieurs parties est disposé dans une frette tubulaire métallique 21 s'étendant sur toute sa longueur et rigidifiant l'élément longiforme C.

Ce mode de construction et de liaison permet de réaliser, à l'intérieur du corps tubulaire 20, diverses cloisons ou collerettes, visibles figure 3, telles que
- la collerette 26, servant à l'appui d'un ressort 27 poussant le piston 25 contre le profil en came 6c de la poignée 6,
- une cloison 28 servant à l'appui d'une douille d'armement 29, soumise en permanence à l'action d'un ressort 30 en appui sur une autre collerette 32,
- un palier 33 dans lequel est emprisonnée la collerette d'une douille de calage 34,
- une collerette 35 servant à l'appui du coulisseau-magasin 22 lorsqu'il est en position de repos,
- et une cloison 31 pénétrant dans la cage 23 et servant à l'appui de son ressort de rappel 38.

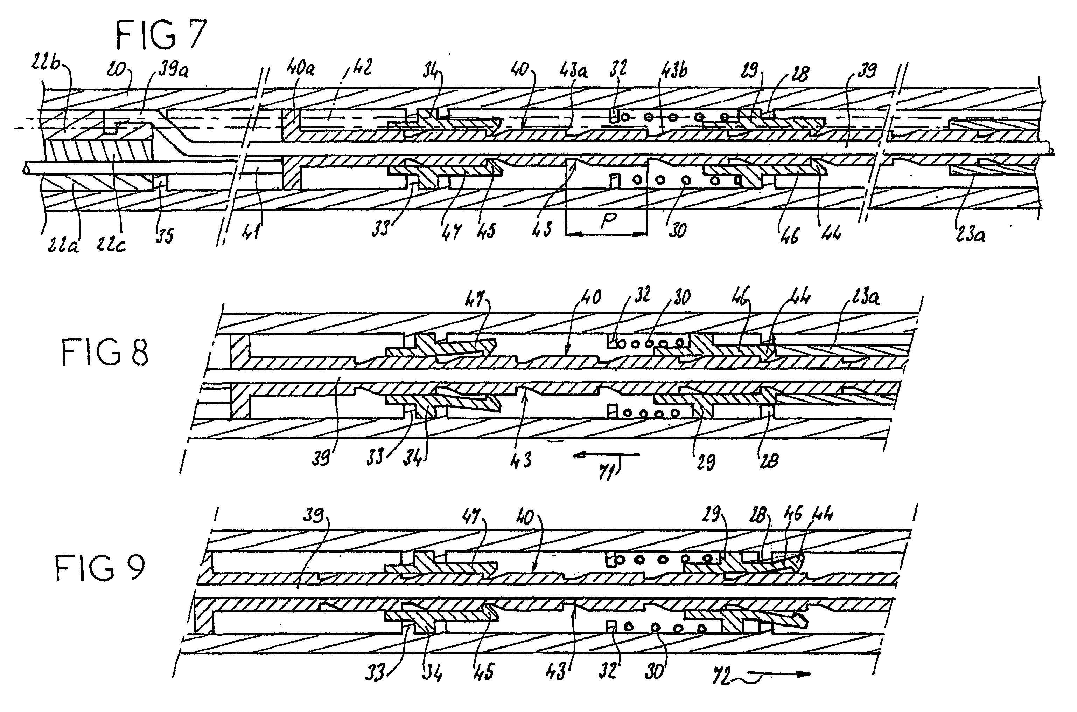

Le piston 25 est traversé axialement par une tige de commande 36 qui traverse également le tube 24, de liaison du piston avec la cage 23, et pénètre dans la cage, où son extrémité antérieure reçoit par une collerette 37 la poussée du ressort 38. La tige 36 est prolongée par une tige axiale 39, de plus petit diamètre qu'elle et dont l'extrémité antérieure 39a, coudée et contrecoudée, est liée au coulisseau 22, comme montré à la figure 7.

La cage 23, qui est montée coulissante dans le corps tubulaire 20, se prolonge sur l'avant par un embout tubulaire 23a, de plus petit diamètre qu'elle. L'alésage interne de cet embout sert au guidage d'un arbre cylindrique 40, traversé axialement par la tige 39 et dénommé arbre creux à gorges. Enfin, la cage 23 est solidaire de la tige poussoir 42 traversant le coulisseau 22.

Comme montré plus en détails figures 7 à 9 qui sont des vues en coupe par un plan décalé de 90° par rapport au plan de coupe de la figure 3, l'arbre 40 s'étend au-delà du palier 33 pour la douille de calage 34 et comporte une extrémité en forme de piston 40a qui coulisse dans le corps tubulaire 20 et contribue à son guidage en translation. Cette extrémité 40a est solidaire d'une tige de butée 41 qui s'étend longitudinalement et pénètre dans le coulisseau 22. L'arbre 40 comporte des gorges extérieures 43. Dans chacune de ces gorges, le bord avant 43a est droit, c'est-à-dire situé dans un plan diamétral, tandis que le bord arrière 43b est pentu et va en s'éloignant du bord droit en partant du fond de la gorge. Ces gorges sont réparties sur l'arbre 40 avec un pas P de valeur égale à la longueur L (figure 1) de la barre d'arrêt 3 d'une attache A. Elles sont destinées à coopérer avec des crans, respectivement 44 et 45, de languettes élastiques opposées, respectivement 46, 47, solidaires, respectivement de la douille d'armement 29 et de la douille de calage 34.

Comme montré plus en détails aux figures 4 à 6, le coulisseau-magasin 22 est formé par deux coquilles en matière plastique 22a, 22b, s'assemblant, par collage ou soudure, selon leur plan médian longitudinal et vertical, en enserrant une plaquette intercalaire 22c.

La coquille 22a est solidaire, à son extrémité, d'une aiguille creuse et fendue 50 et comporte, près de ses bords, deux rainures longitudinales, à savoir, une rainure 52, servant de logements aux barres d'ancrage 2 des attaches A, et une rainure 53 servant de logements aux barres d'arrêt 3 des mêmes attaches. Ces deux rainures sont dans un même plan vertical, distinct de celui passant par l'aiguille, et celle 53 débouche librement de l'extrémité antérieure de la coquille 22a, tandis que celle 52 vient mourir sur un plan incliné 54, visible figure 6. Ce dernier favorise le déplacement transversal de la barre d'ancrage 2 de la première attache jusqu'à ce que cette barre vienne derrière et dans l'axe longitudinal de l'aiguille creuse 50, dans un logement d'accueil 55, comme montré figure 4.

La coquille 22a comporte encore, à côté de l'aiguille 50, une nervure 51 saillant vers le bas et présentant, au-dessus de la trajectoire de sortie d'une barre d'arrêt de la rainure 53, une face pentue 51 a.

La figure 6, qui représente l'appareil en position de repos, montre que, dans cette position, la tige du poussoir 42 est en retrait de l'extrémité postérieure de l'aiguille 50 pour dégager le logement d'accueil 55 ménagé dans l'autre coquille 22b.

La figure 4 montre que le logement d'accueil 55 pour la barre d'ancrage 2 de la première attache est prolongé vers le bas par une face inclinée 56 de soutien de la barrette de liaison 4 de l'attache. Cette coquille 22b comporte également une rainure semi cylindrique 57 qui, avec une rainure complémentaire 58 ménagée dans la plaquette 22c, forme un canal de guidage pour la tige poussoir 42. Ce canal est coaxial à l'aiguille 50. Par sa partie arrière, cette coquille 22b est liée à l'extrémité 39a de la tige de commande 39, tandis que son extrémité avant est munie, dans sa partie venant sous l'aiguille, d'un doigt transversal 59 saillant en direction de l'autre coquille, présentant une face pentue 59a et dont l'utilité sera précisée ultérieurement.

La plaquette intercalaire 22c a une largeur lui permettant de s'insérer entre les rainures 52 et 53 de la coquille 22a et une épaisseur lui permettant de former, avec cette coquille 22a, un couloir 60, visible figure 5, permettant de libérer le passage pour les barrettes de liaison 4 des attaches.

Le coulisseau-magasin 22, ainsi formé, a une longueur lui permettant de stocker plusieurs attaches A et, par exemple, dix attaches.

Lorsque l'appareil est au repos, la poignée 6 est dans la position représentée à la figure 2, avec la tige axiale de commande 36 en appui sur l'extrémité inférieure du levier 8 et le piston 25 en appui sur les rampes 6c de la poignée 6. Le coulisseau-magasin 22 est en position rétractée dans le corps tubulaire 20. L'aiguille 50 est rétractée dans le corps et la tige poussoir 42 qui dégage le logement d'accueil 55 n'exerce aucun effort sur la barre d'ancrage 2 disposée dans ce logement. De même, les barres d'arrêt 3 des attaches A en attente dans le magasin sont en contact les unes avec les autres, tandis que la barre d'arrêt de la dernière attache est à proximité, avec ou sans contact, de l'extrémité de la tige de butée 35, mais sans effort significatif.

Il en résulte qu'aucune des attaches n'est soumise à un quelconque effort pouvant, dans le temps, la faire fluer, la déformer et perturber son transfert.

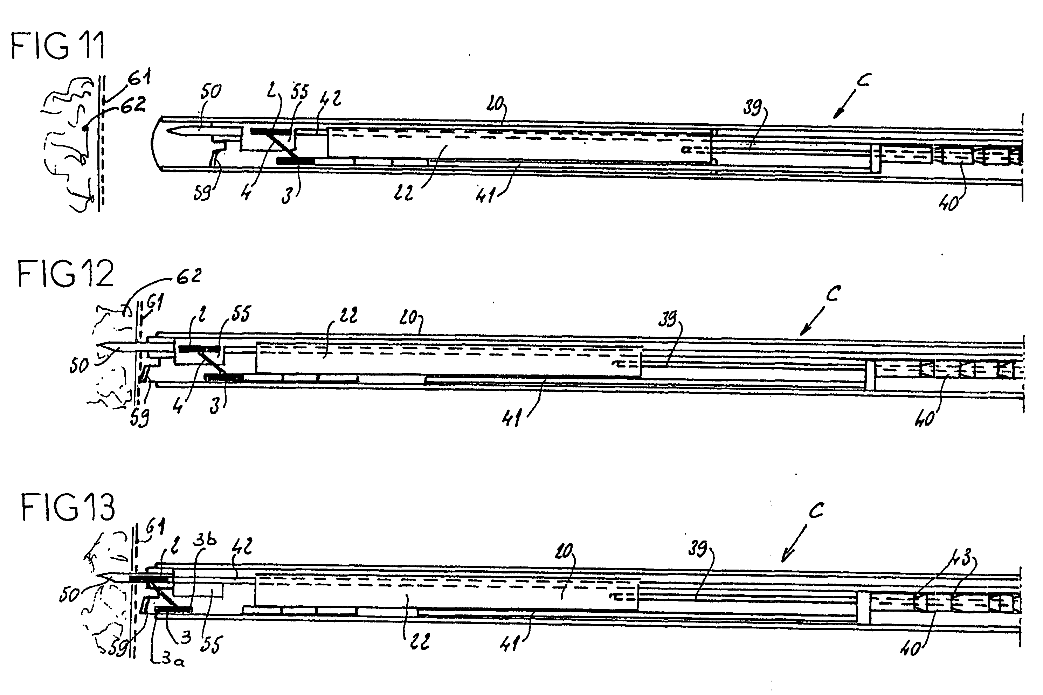

En utilisation, l'élément longiforme C de l'appareil est introduit, avec l'aiguille 50 rentrée, dans un trocart. A partir de là, il peut être utilisé de deux façons :
- l'engagement est effectué jusqu'au contact des tissus, puis l'aiguille est sortie par actionnement de la poignée 6, pour traverser ceux-ci, comme montré figure 12,
- l'engagement est effectué en arrêtant l'extrémité du corps 20 à proximité et à distance de la paroi à renforcer, comme montré figure 11, puis en commandant la sortie de l'aiguille sans la faire pénétrer dans les tissus.

Cette dernière solution est préférée, car elle permet de visualiser la position de l'aiguille 50 par rapport aux zones devant recevoir une attache et d'adapter cette position en faisant pivoter l'élément C par rapport au corps de préhension. C'est donc quand l'aiguille 50 est bien positionnée qu'elle est introduite, par déplacement de l'ensemble de l'appareil, dans les deux tissus 61, 62 dont il faut assurer la liaison, et par exemple, un renfort textile 61 et une paroi abdominale 62,

Pour faire sortir l'aiguille 50, l'opérateur presse la poignée 6 afin qu'elle pivote en rentrant dans le corps. Sur une première partie de la course l'extrémité inférieure du levier 8 se déplace davantage que le piston 25, de sorte que, par la tige 36, le coulisseau 22 se déplace en faisant saillir l'aiguille 50, hors du corps 20.

La figure 10 montre que, quand l'aiguille 50 est complètement sortie, la poignée arrive sensiblement à mi course et vient en contact contre le ressort en épingle 10. Par l'effort résistant qu'il fournit sur la poignée 6, ce ressort indique à l'opérateur cette position et que la continuation du mouvement peut entraîner la distribution d'une attache. Cela permet, tout en maintenant l'effort sur la poignée, de faire éventuellement pivoter l'élément 20 pour l'adapter aux besoins de la pose. A partir de cette position, les doigts 6a de la fourchette exercent sur les tenons 8a un effort suffisant pour maintenir le levier 8 dans sa position, mais sans générer son déplacement.

Quand le pivotement de la poignée 6 est continué, ce sont les seconds moyens de commande qui agissent et qui ne commandent que le déplacement du piston 25, donc du poussoir 42, ce déplacement s'effectuant par rapport au coulisseau-magasin 22 qui reste temporairement fixe.

La tige poussoir 42 est donc déplacée longitudinalement afin que son extrémité vienne en contact avec la partie arrière de la barre d'ancrage 2 de l'attache A disposée dans le logement d'accueil 55, et pousse cette barre d'ancrage dans l'aiguille 50, comme montré à la figure 13.

Le déplacement de la barre d'ancrage 2 tire la barrette de liaison 4 et la barre d'arrêt 3 de l'attache en cours de distribution. L'extrémité aval 3a de la barre d'arrêt 3 vient en butée contre le doigt 59, ce qui provoque le basculement vers le haut de cette barre jusqu'à la redresser parallèlement au renfort 61. Durant ce mouvement, son extrémité amont 3b rencontre la face pentue 51a de la nervure 51 qui chasse transversalement cette extrémité, afin que, lors du redressement de la barre, cette extrémité ne vienne pas buter contre l'aiguille mais au contraire s'en éloigne.

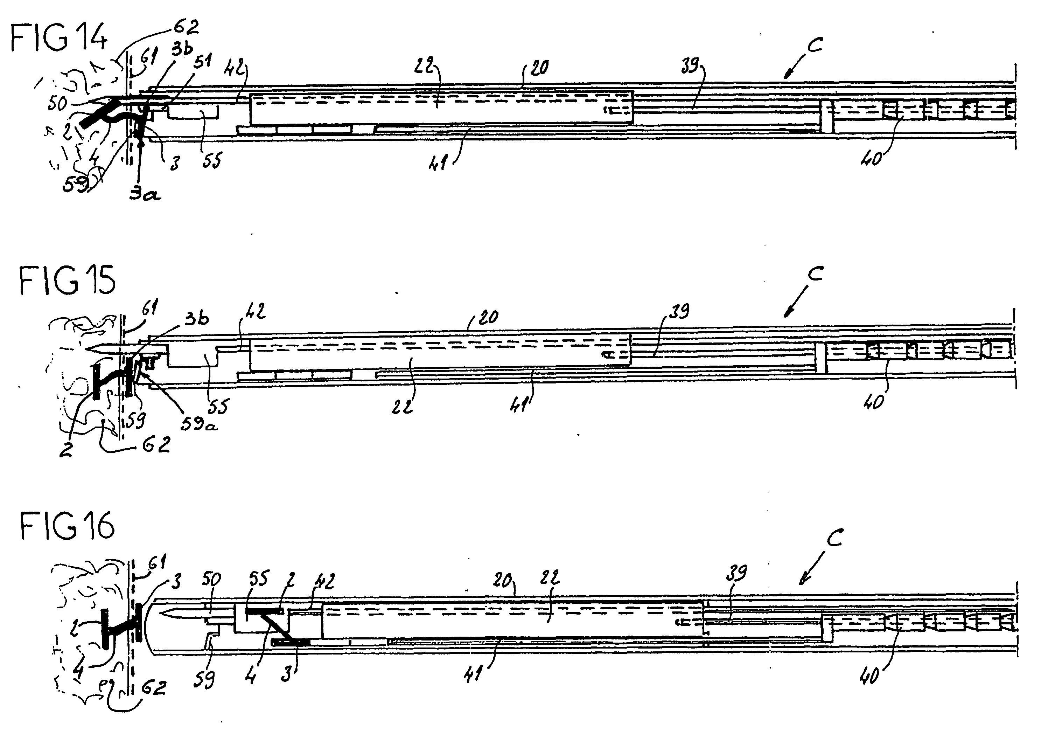

Durant ce mouvement, la barre d'ancrage 2 avance dans l'aiguille. Quand la barrette de liaison 4 échappe de celle-ci, la barre 2 peut commencer à basculer avec l'aide de la barre d'arrêt 3. En effet, par la barrette de liaison 4, le mouvement organisé de la barre d'arrêt 3 a pour conséquence de retenir la barre d'ancrage 2 qui est ainsi forcée à se redresser et à s'ancrer dans les tissus biologiques 62, comme montré à la figure 14. Pendant ou en fin de redressement de la barre d'ancrage, la barre d'arrêt 3 qui est sensiblement parallèle au renfort textile 61, glisse, par son extrémité aval 3a, sur la face pentue 59a du doigt 59 qui la chasse transversalement et la libère.

A la fin de ce mouvement, et comme montré à la figure 15, le relâchement de l'action sur la poignée 6, permet aux ressorts 27 et 38 de rappeler, respectivement, le coulisseau-magasin 22 et la tige poussoir 42. C'est après ce relâchement de la poignée que l'appareil est extrait du corps humain, comme montré figure 16.

Il ressort de ce qui précède que, pendant la distribution de l'attache A, les moyens de distribution mis en oeuvre permettent, à sa barre d'ancrage et à sa barre d'arrêt, de se redresser pour assurer une parfaite retenue de ces tissus.

On notera que, pendant la phase de distribution de la première attache A, et comme montré aux figures 13 à 15, le logement d'accueil 55 est obturé par la tige poussoir 42 et que les attaches en attente conservent leur position, puisque, durant ce déplacement, elles ne sont soumises à aucun effort par la barre de butée 41. En effet, le déplacement du coulisseau-magasin 22 vers l'avant de l'appareil, emmène avec lui les attaches en stock et les éloigne donc de l'extrémité libre de la tige de butée 41, qui n'est mise en mouvement que sur la fin du déplacement de la tige poussoir 42.

Comme montré à la figure 8, ce déplacement est assuré par contact de l'extrémité de l'embout 23a de la cage 23, sur l'extrémité des languettes 46 de la douille d'armement 29. En raison du déplacement de cette douille 29, dont les crans restent engagés dans une gorge 43 de l'arbre 40, cet arbre est soumis à un effort longitudinal qui, par les bords pentus 43b de la gorge en prise avec les crans 45 de la douille de calage 34, provoque l'écartement des languettes 47 portant ces crans 45 et permet le déplacement longitudinal de cet arbre, dans le sens de la flèche 71 de figure 8. Ce déplacement est effectué jusqu'à ce que les crans 45 tombent dans la gorge 43 suivante avec l'aide du rappel élastique des languettes 46. Cette position correspond à la fin du déplacement de la cage 23 par le piston 25 en appui sur le profil en came 6c de la poignée.

Il résulte de cela que l'extrémité de la tige de butée 41 a été avancée d'un pas par rapport à la position initiale qu'elle occupait. De la sorte, lors du recul du coulisseau-magasin 22, la barre d'arrêt 3 de la dernière attache vient en contact avec l'extrémité de la barre de butée 41 et retient la rangée d'attaches, tandis que le coulisseau termine sa rentrée dans le corps 20.

En fin de rentrée du coulisseau, la barre d'ancrage 2 de la première attache vient en contact avec la rampe 54, montrée figure 6, rampe qui assure son transfert dans le logement d'accueil 55.

En raison des jeux fonctionnels dans l'appareil et de l'absence de moyens à ressort poussant les attaches vers la sortie du magasin, la barre d'ancrage 2 de la première attache est stockée sans contrainte dans le logement d'accueil 55, tandis que sa barre d'arrêt 3, qu'elle soit ou non en appui sur les barres d'arrêt des autres attaches A stockées, est sans contrainte ou soumise à un effort d'appui non suffisant pour la déformer, puisque, même si la barre d'arrêt 3 de la dernière attache reste en contact avec la barre de butée 41, c'est sans contrainte.

La figure 9 montre que le relâchement de la poignée 6 provoquant le retrait du coulisseau dans le corps tubulaire 20 de l'élément longiforme C, éloigne l'embout 23a de la douille d'armement 29 et permet à celle-ci, sous la poussée de son ressort 30, de se déplacer dans le sens de la flèche 72, et cela d'autant plus aisément que, sous l'effort qu'elle subit, ses crans 44 rencontrant la face pentue 43b de la gorge 43 dans laquelle ils sont, provoquent l'écartement des languettes 46 jusqu'à leur retombée dans la gorge suivante, en armant le dispositif pour un prochain déplacement de la tige de butée.

L'appareil, qui a été décrit dans le cadre de son application au stockage, à la distribution et à la pose d'attaches chirurgicales en matériau biorésorbables à faible élasticité, peut très bien distribuer des attaches réalisées dans un matériau ayant de meilleures caractéristiques mécaniques, puisque le stockage, la distribution et la pose s'effectue dans des conditions plus défavorables.

A titre d'exemple, pour un appareil dont le diamètre extérieur de l'élément longiforme C est de 10 millimètres, distribuant des attaches en "I" couché ayant une hauteur de 7 millimètres, et des barres d'ancrage et d'arrêt ayant une longueur de 7 millimètres, la course de déplacement du coulisseau-magasin est de l'ordre de 13 millimètres, alors que la course du poussoir d'éjection 42 est de l'ordre de 30 millimètres avec un déplacement de l'arbre 40 portant les gorges 43 sur les 7 derniers millimètres de la course.

## Revendications

1. Appareil de stockage, de distribution et de pose d'attaches chirurgicales (A) en "I" couché comprenant un corps de poignée (B) équipé de premiers et seconds moyens de commande d'une poignée (6) et d'un embout (13), et un élément longiforme (C), rapporté et fixé sur l'embout, appareil dans lequel l'élément longiforme (C) est composé d'un corps (20) de forme générale tubulaire contenant :
- à proximité de son extrémité libre, un coulisseau-magasin (22) contenant lui-même des attaches (A) en "I" couché ayant chacune une barre d'ancrage (2), une barre d'arrêt (3), et une barrette de liaison (4), ledit coulisseau (22) étant, d'une part, déplaçable dans l'élément longiforme par une tige longitudinale (39) actionnable par les premiers moyens de commande de la poignée (6a, 8a, 8), et, d'autre part, solidaire d'une aiguille creuse (50), fendue et longitudinale, saillant de son extrémité libre et pouvant saillir de cet élément longiforme (C),
- et, dans son corps tubulaire (20), d'une part, des moyens de distribution une à une des attaches (A) stockées, cette distribution comprenant le transfert de la barre d'ancrage (2) de la première attache dans un logement d'accueil (55) disposé dans le prolongement de l'aiguille creuse (50), et, d'autre part, un poussoir d'éjection (42), déplaçable par les seconds moyens de commande (25) de la poignée et disposé dans l'alignement de l'aiguille creuse (50) pour pousser dans celle-ci la barre d'ancrage (2) de la première attache (A) **caractérisé en ce que** le logement d'accueil (55) du coulisseau-magasin (22) contient, au repos, la barre d'ancrage (2) de la première attache A en attente de pose, attache dont la barre d'arrêt (3), disposée à l'extrémité du magasin, est libre de tout effort de poussée, tandis que ce coulisseau-magasin (22) comporte :
- au-dessous de l'aiguille (50) et dans la trajectoire de sortie du magasin de la dite barre d'arrêt (3), un doigt transversal (59) apte, lors de la distribution de l'attache par déplacement de sa barre d'ancrage (2) au moyen du poussoir (42), à recevoir l'appui de l'extrémité aval (3a) de cette barre d'arrêt pour la forcer à basculer en soulevant son extrémité amont (3b),
- à coté de l'aiguille (50), une nervure (51) saillant vers le base et apte à guider le basculement et le déplacement de l'extrémité amont (3b) de la barre d'arrêt (3), pour que cette dernière ne vienne pas buter contre l'aiguille,
- et, sur le doigt transversal (59), une face pentue (59a) chassant, transversalement et hors de ce doigt, l'extrémité aval (3a) de la barre d'arrêt (3).

2. Appareil de stockage, de distribution et de pose d'attaches selon la revendication 1, **caractérisé en ce que**, au repos, l'extrémité antérieure du poussoir (42) est espacée de l'extrémité arrière de l'aiguille (50) pour former le logement d'accueil (55) contenant la barre d'ancrage (2) de la première attache (A),

3. Appareil de stockage, de distribution et de pose d'attaches selon la revendication 1, **caractérisé en ce que** la barre d'arrêt (3) de la première attache est, dans le magasin, au contact des barres d'arrêt des autres attaches (A) stockées, et la barre d'arrêt (3) de la dernière attache est au contact, mais sans effort significatif, avec l'extrémité d'une tige de butée (41), déplaçable pas à pas, cette tige de butée (41) étant reliée à des moyens (40, 29, 34) aptes, en fin de mouvement de distribution de la première attache dans les tissus (61, 62) et par le mouvement des seconds moyens de commande, à la déplacer de la longueur d'une barre d'arrêt (3) d'une attache, afin que, lors du retour du coulisseau-magasin (22) dans sa position de départ et de repos dans le corps tubulaire (20), le contact entre l'extrémité de cette tige de butée (41) et la barre d'arrêt (3) de la dernière attache provoque le déplacement de la rangée d'attaches (A) dans le magasin, et le transfert de la barre d'ancrage (2) de la première attache dans le logement d'accueil (55).

4. Appareil de stockage, de distribution et de pose d'attaches selon la revendication 3, **caractérisé en ce que** la tige de butée (41), montée coulissante dans le corps du coulisseau-magasin (22), est liée par son extrémité arrière à un arbre (40), ledit arbre étant :
- monté coulissant sur une tige axiale (39) s'étendant entre les premiers moyens de commande (8) de la poignée et le coulisseau (22), dont cette tige est solidaire,
- muni de gorges (43), espacées d'un pas égal à la longueur de la barre d'arrêt (3) d'une attache (A) et chacune munie d'un bord avant droit (43a) et d'un bord arrière pentu (43b),
- calé en position de travail par des languettes élastiques (47) crantées saillant d'une douille de calage (34) liée au corps tubulaire (20) et dont les crans (45) pénètrent dans l'une des gorges (43) disposée sur la partie antérieure de l'arbre à gorges (40),
- lié à une douille d'armement (29), disposée en arrière de la douille de calage (34), montée coulissante dans le corps tubulaire (20) à l'encontre de moyens de rappel (30) la plaquant contre une collerette interne (28) de ce corps, cette liaison étant assurée par pénétration de languettes crantées (46) solidaires de cette douille d'armement dans l'une des gorges (43) de l'arbre à gorges (40),
- et associé à des moyens de déplacement constitués par un corps longitudinal (23a) actionnable par les seconds moyens de commande (25, 6c) et venant, en fin de course de distribution et de pose d'une attache, pousser la douille d'armement (29) de la longueur d'une barre d'arrêt (3) pour déplacer l'arbre à gorges (40), en provoquant l'écartement des languettes (47) de la douille de calage (34) jusqu'à pénétration de leurs crans dans la gorge suivante, et l'avancement de la tige de butée (41) de la valeur d'une barre d'arrêt (3) d'une attache.

5. Appareil de stockage, de distribution et de pose d'attaches selon la revendication 4, **caractérisé en ce que** le corps longitudinal (23a) provoquant le déplacement de la douille d'armement (29) est constitué par un embout tubulaire (23a) entourant l'arbre (40) à gorges et saillant de l'extrémité avant d'une cage (23) solidaire du poussoir (42), ladite cage, contenant les moyens de rappel (38) de la tige axiale (39) du coulisseau (22), étant solidaire d'un piston de commande (25) pénétrant dans le corps (6) de poignée et coopérant avec les seconds moyens de commande (6c)

6. Appareil de stockage, de distribution et de pose d'attaches selon la revendication 1, **caractérisé en ce que** l'extrémité arrière de la tige (39) liée au coulisseau-magasin (22) est en appui sur un levier (8) articulé dans le corps de poignée (6) et déplaçable par une fourchette à deux doigts (6a) de la poignée (6) articulée dans ce corps, tandis que le piston (25), solidaire de la cage (23) et du poussoir (39), est en appui sur un profil en came (6c) ménagé sur la poignée (6).

7. Appareil de stockage, de distribution et de pose d'attaches selon la revendication 1, **caractérisé en ce que** le coulisseau-magasin (22) est formé par deux coquilles en matière plastique (22a, 22b) s'assemblant selon leur plan médian, longitudinal et vertical, la coquille (22a) solidaire de l'aiguille creuse et fendue (50) comportant deux rainures longitudinales (53, 52), disposées près de ses bords et aptes à recevoir, l'une, les barres d'arrêt (3) des attaches, l'autre, les barres d'ancrage (2), la rainure (53) pour les barres d'arrêt (3) débouchant librement de l'extrémité avant du coulisseau (22) alors que la rainure (52) pour les barres d'ancrage (2) débouche sur un plan incliné (54) de guidage en direction de l'aiguille (50), tandis que l'autre coquille (22b) comporte un canal (57, 58), longitudinal et coaxial à l'aiguille (50), pour le poussoir d'éjection (42) et, dans le logement (55) d'accueil de l'aiguille (A), un plan de repos (56) incliné verticalement, pour la barrette de liaison (4) d'une attache.

8. Appareil de stockage, de distribution et de pose d'attaches selon la revendication 7, **caractérisé en ce que** le canal pour le poussoir d'éjection (42) est formé par une rainure (57) ménagée dans la coquille (22b) correspondante et par une rainure (58) ménagée dans une plaquette (22c) rapportée à l'intérieur de ladite coquille (22b), ladite plaquette (22c) ayant une largeur lui permettant de s'insérer entre les rainures (52, 53) ménagées dans l'autre coquille (22a), respectivement, pour les barres d'ancrage (2) et pour les barres d'arrêt (3), et une épaisseur lui permettant de délimiter avec cette autre coquille (22a), un couloir (60) pour le passage des barrettes de liaison (3) des attaches.

9. Appareil de stockage, de distribution et de pose d'attaches selon la revendication 1, **caractérisé en ce que** le corps longiforme (C) est composé de plusieurs éléments en matière plastique assemblés entre eux et disposés dans une frette tubulaire métallique (21).

## Patentansprüche

1. Vorrichtung zur Verwahrung, Ausgabe und Anbringung von chirurgischen Anbringelementen (A) in Form eines liegenden "I", die einen Griffkörper (B), der mit ersten und zweiten Griffbetätigungsmitteln (6) und einem Aufsatz (13) ausgestattet ist, und ein längliches Element (C) aufweist, das auf dem Aufsatz fixiert bereitgestellt ist, wobei die Vorrichtung, in der ein längliches Element (C) einen Körper (20) von allgemeiner röhrenförmiger Gestalt aufweist, Folgendes aufweist, nämlich:
- in der Nähe ihres freien Endes einen Magazinschieber (22), der selbst Anbringelemente (A) in Form eines liegenden "I" aufweist, wovon jedes einen Verankerungsstab (2), einen Arretierstab (3) und einen Verbindungssteg (4) aufweist, wobei der Schieber (22) einerseits in dem länglichen Element über einen länglichen Stift (39) verschiebbar ist, der durch die ersten Griffbetätigungsmittel (6a, 8a, 8) betätigbar ist, und andererseits mit einer geschlitzten und länglichen Hohlnadel (50) verbunden ist, die an seinem freien Ende hervorsteht und über das längliche Element (C) hervorragen kann,
- und in ihrem röhrenförmigen Körper (20) einerseits Mittel zur einzelnen Ausgabe der verwahrten Anbringelemente (A), deren Ausgabe die Überführung des Verankerungsstabes (2) des ersten Anbringelementes in einen Aufnahmeraum (55) umfasst, der in der Verlängerung der Hohlnadel (50) angeordnet ist, und andererseits eine Ausschubvorrichtung (42), die durch die zweiten Griffbetätigungsmittel (25) verschiebbar und in der Ausrichtung der Hohlnadel (50) angeordnet ist, um in diese den Verankerungsstab (2) des ersten Anbringelementes (A) zu drücken, **dadurch gekennzeichnet, dass** der Aufnahmeraum (55) des Magazinschiebers (22) in Ruhestellung den Verankerungsstab (2) des ersten Anbringelementes (A) in Warteposition aufweist, wobei der Arretierstab (3) des Anbringelementes, der am Ende des Magazins angeordnet ist, frei von jeglicher Schubkraft ist, wohingegen der Magazinschieber (22) Folgendes aufweist, nämlich:
- unter der Nadel (50) und in der Auslassbahn des Magazins für den Arretierstab (3) einen quer verlaufenden Zapfen (59), der geeignet ist, während der Ausgabe des Anbringelementes durch Verschieben seines Verankerungsstabes (2) mit Hilfe des Ausschiebers (42) die Lagerung des stromabwärts gelegenen Endes (3a) des Arretierstabes zu übernehmen, um ein Kippen zu bewirken, indem sein stromabwärts gelegenes Ende (3b) angehoben wird,
- in der Nähe der Nadel (50) einen Steg (51), der zur Basis hin vorragt und dazu geeignet ist, das Umkippen und das Verschieben des stromaufwärts gelegenen Endes (3b) des Arretierstabes (3) zu lenken, damit Letzterer nicht gegen die Nadel stößt,
- und auf dem quer verlaufenden Zapfen (59) eine abschüssige Seite (59a), die das stromabwärts gelegene Ende (3a) des Arretierstabes (3) schräg und von dem Zapfen wegdrückt.

2. Vorrichtung zur Verwahrung, Ausgabe und Anbringung von Anbringelementen nach Anspruch 1, **dadurch gekennzeichnet, dass** in Ruhestellung das Vorderende des Ausschiebers (42) von dem hinteren Ende der Nadel (50) beabstandet ist, um den Aufnahmeraum (55) zu bilden, der den Verankerungsstab (2) des ersten Anbringelementes (A) aufweist.

3. Vorrichtung zur Verwahrung, Ausgabe und Anbringung von Anbringelementen nach Anspruch 1, **dadurch gekennzeichnet, dass** der Arretierstab (3) des ersten Anbringelements in dem Magazin in Kontakt mit Arretierstäben anderer verwahrter Anbringelemente (A) steht, und der Arretierstab (3) des letzten Anbringelementes steht in Kontakt, jedoch ohne wesentlichen Druck, mit dem Ende einer Anschlagstange (41), die stufenweise verschiebbar ist, wobei die Anschlagstange (41) mit Mitteln (40, 29, 34) verbunden ist, die dazu geeignet sind, am Ende der Bewegung zur Verteilung des ersten Anbringelementes in die Gewebe (61, 62) und durch Bewegung der zweiten Betätigungsmittel dieses für die Länge eines Arretierstabes (3) eines Anbringelementes zu verschieben, damit, bei der Rückkehr des Magazinschiebers (22) in seine Ausgangs- und Ruheposition im röhrenförmigen Körper (20), der Kontakt zwischen dem Ende der Anschlagstange (41) und der Arretierstange (3) des letzten Anbringelements die Verschiebung der Reihe von Anbringelementen (A) in dem Magazin und die Übertragung des Verankerungsstabes (2) des ersten Anbringelements in dem Aufnahmeraum (55) bewirkt.

4. Vorrichtung zur Verwahrung, Ausgabe und Anbringung von Anbringelementen nach Anspruch 3, **dadurch gekennzeichnet, dass** die Anschlagstange (41), die in dem Körper des Magazinschiebers (22) verschiebbar angebracht ist, über ihr hinteres Ende mit einer Welle (40) verbunden ist, wobei diese Welle:
- verschiebbar auf einer Achsstange (39) angebracht ist, die sich zwischen den ersten Griffbetätigungsmitteln (6) und dem Schieber (22) erstreckt, mit dem diese Stange verbunden ist,
- mit Vertiefungen (43) versehen ist, die einen Abstand zueinander aufweisen, der der Länge des Arretierstabes (3) eines Anbringelementes (A) entspricht, wobei jede Vertiefung (43) mit einer vertikalen Vorderkante (43a) und einer abschüssigen hinteren Kante (43b) versehen ist,
- in Arbeitsposition durch verrastete elastische Federn (47) festgeklemmt ist, die von einer Positionierhülse (34) hervorstehen, die mit dem röhrenförmigen Körper (20) verbunden ist, und deren Rasten (45) in eine der Vertiefungen (43) eindringen, die auf dem Vorderteil der mit Vertiefungen versehenen Welle (40) angeordnet ist,
- mit einer Spannhülse (29) verbunden ist, die hinter der Positionierhülse (34) angeordnet ist, die verschiebbar in dem röhrenförmigen Körper (20) gegen ein Rückstellmittel (30) angebracht ist, das gegen einen inneren Flansch (28) des Körpers drückt, wobei diese Verbindung durch das Eindringen von Rastfedern (46) gesichert wird, die mit der Spannhülse in einer der Vertiefungen (43) der mit Vertiefungen versehenen Welle (40) verbunden sind,
- ferner mit Verschiebemitteln verbunden ist, die durch einen länglichen Körper (23a) gebildet werden, der durch die zweiten Betätigungsmittel (25, 6c) betätigbar ist, und am Ende der Ausgabe und Anbringung eines Anbringelements die Spannhülse (29) über die Länge eines Arretierstabes (3) verrückt, um die mit Vertiefungen versehene Welle (40) zu verschieben, indem das Auseinandergehen der Federn (47) der Positionierhülse (34) bis zum Eindringen ihrer Rasten in die nachfolgende Vertiefung und das Fortschreiten der Anschlagstange (41) um den Wert eines Arretierstabes (3) eines Anbringelementes bewirkt wird.

5. Vorrichtung zur Verwahrung, Ausgabe und Anbringung von Anbringelementen nach Anspruch 4, **dadurch gekennzeichnet, dass** der längliche Körper (23a), der die Verschiebung der Spannhülse (29) bewirkt, durch einen röhrenförmigen Aufsatz (23a) gebildet ist, der die mit Vertiefungen versehene Welle (40) umgibt, und am Ende eines Gehäuses (23) hervorsteht, das mit dem Ausschieber (42) verbunden ist, wobei das Gehäuse, das die Rückstellmittel (38) von der Achsstange (39) des Schiebers (22) aufweist, mit einem Betätigungskolben (25) verbunden ist, der in den Griffkörper (6) eindringt und mit den zweiten Betätigungsmitteln (6c) zusammenwirkt.

6. Vorrichtung zur Verwahrung, Ausgabe und Anbringung von Anbringelementen nach Anspruch 1, **dadurch gekennzeichnet, dass** das hintere Ende der Stange (39), die mit dem Magazinschieber (22) verbunden ist, auf einem Hebel (8) aufliegt, der in dem Griffkörper (6) gelenkig angebracht ist, und über eine zweifingrige Gabel (6a) des Griffes (6) verschiebbar ist, die in dem Körper gelenkig angebracht ist, während der mit dem Gehäuse (23) und der Schubstange (39) verbundene Kolben (25) auf einem Nockenprofil (6c) aufliegt, das auf dem Griff (6) angebracht ist.

7. Verfahren zur Verwahrung, Ausgabe und Anbringung von Anbringelementen nach Anspruch 1, **dadurch gekennzeichnet, dass** der Magazinschieber (22) durch zwei Schalen aus Kunststoffmaterial (22a, 22b) gebildet ist, die entlang ihrer Mittel-, Längs- und Vertikalebene zusammengesetzt sind, wobei die Schale (22a), die mit der geschlitzten Hohlnadel (50) verbunden ist, zwei Längsrinnen (53, 52) aufweist, die in der Nähe ihrer Kanten angeordnet sind und dazu geeignet sind, einerseits die Arretierstäbe (3) der Anbringelemente, und andererseits die Verankerungsstäbe (2) aufzunehmen, wobei die Rinne (53) für die Arretierstäbe (3) vorgesehen ist, die frei an dem Ende vor dem Schieber (22) herausgefahren kommen, wohingegen die Rinne (52) für die Verankerungsstäbe (2) vorgesehen ist und in eine geneigte Ebene zur Führung in Richtung der Nadel (50) mündet, während die andere Schale (22b) einen längs und koaxial zur Nadel (50) verlaufenden Kanal (57, 58) für die Ausschubvorrichtung (42) und in dem Aufnahmeraum (55) für die Nadel (A) eine vertikal geneigte Rastfläche (56) für den Verbindungssteg (4) eines Anbringelements aufweist.

8. Vorrichtung zur Verwahrung, Ausgabe und Anbringung von Anbringelementen nach Anspruch 7, **dadurch gekennzeichnet, dass** der Kanal für die Ausschubvorrichtung (42) durch eine Rinne (57), die in der entsprechenden Schale (22b) angebracht ist und durch eine Rinne (58) gebildet ist, die in einer Leiste (22c) angebracht ist, die innen auf die Schale (22b) aufgesetzt ist, wobei die Leiste (22c) eine Breite hat, die es ihr ermöglicht, sich zwischen die Rinnen (52, 53) zu schieben, die in der anderen Schale (22a) für die Verankerungsstäbe (2) bzw. für die Arretierstäbe angebracht sind, und die eine Dicke hat, die es ihr ermöglicht, mit der anderen Schale (22a) einen Gang (60) für das Hindurchtreten der Verbindungsstege (4) der Anbringelemente zu begrenzen.

9. Vorrichtung zur Verwahrung, Ausgabe und Anbringung von Anbringelementen nach Anspruch 1, **dadurch gekennzeichnet, dass** der längliche Körper (C) aus mehreren Elementen aus Kunststoffmaterial zusammengesetzt ist, die zusammengesetzt und in einem rohrförmigen Metallkörper (21) angeordnet sind.

## Claims

1. Appliance for storing, distributing and placing couched I-shaped surgical fasteners (A), comprising a handgrip body (B) equipped with first and second controls on a handgrip (6) and with an end-piece (13), and an elongate element (C), attached and fixed to the end-piece, in which appliance the elongate element (C) is made up of a body (20) of tubular overall shape containing:
- near its free end, a magazine slide (22) itself containing couched I-shaped fasteners (A) each having an anchoring bar (2), a catching bar (3) and a connecting strip (4), the said slide (22) being, on the one hand, movable in the elongate element by a longitudinal rod (39) which can be actuated by the first controls on the handgrip (6a, 8a, 8) and, on the other hand, secured to a split and longitudinal hollow needle (50) projecting from its free end and able to project from this elongate element (C),
- and, in its tubular body (20), on the one hand, means for distributing the stored fasteners (A) one by one, this distribution involving the transferring of the anchoring bar (2) of the first fastener into an accommodating housing (55) arranged in the continuation of the hollow needle (50) and, on the other hand, an ejection plunger (42) which can be moved by the second controls (25) on the handgrip and which is arranged in line with the hollow needle (50) so as to push the anchoring bar (2) of the first fastener (A) thereinto,
**characterized in that** the accommodating housing (55) of the magazine slide (22) contains, at rest, the anchoring bar (2) of the first fastener A awaiting placement, of which fastener the catching bar (3), arranged at the end of the magazine, is free of any thrusting force, while this magazine slide (22) comprises:
- below the needle (50) and in the path via which the said catching bar (3) leaves the magazine, a transverse finger (59) able, as the fastener is distributed by movement of its anchoring bar (2) by means of the plunger (42), to have the downstream end (3a) of this catching bar bear against it to force it to pivot by raising its upstream end (3b),
- beside the needle (50), a downwardly projecting rib (51) able to guide the pivoting and movement of the upstream end (3b) of the catching bar (3) so that the latter does not butt against the needle,
- and, on the transverse finger (59), a sloping face (59a) driving the downstream end (3a) of the catching bar (3) transversely away from this finger.

2. Appliance for storing, distributing and placing fasteners according to Claim 1, **characterized in that**, at rest, the front end of the plunger (42) is spaced away from the rear end of the needle (50) to form the accommodating housing (55) containing the anchoring bar (2) of the first fastener (A).

3. Appliance for storing, distributing and placing fasteners according to Claim 1, **characterized in that** the catching bar (3) of the first fastener is, in the magazine, in contact with the catching bars of the other fasteners (A) stored, and the catching bar (3) of the last fastener is in contact, but without significant force, with the end of a buffer rod (41) which can be moved step by step, this buffer rod (41) being connected to means (40, 29, 34) able, at the end of the movement of distributing the first fastener into the tissues (61, 62), and through movement of the second controls, to move it by the length of one catching bar (3) of one fastener so that, as the magazine slide (22) returns to its starting and rest position in the tubular body (20), contact between the end of this buffer rod (41) and the catching bar (3) of the last fastener causes the row of fasteners (A) to move within the magazine, and causes the anchoring bar (2) of the first fastener to be transferred into the accommodating housing (55).

4. Appliance for storing, distributing and placing fasteners according to Claim 3, **characterized in that** the buffer rod (41), mounted to slide in the body of the magazine slide (22), is connected by its rear end to a shaft (40), the said shaft being:
- mounted to slide on an axial rod (39) extending between the first controls (8) on the handgrip and the slide (22) to which this rod is secured,
- equipped with grooves (43), spaced at a spacing equal to the length of the catching bar (3) of a fastener (A) and each equipped with a straight front edge (43a) and a sloping rear edge (43b),
- held in the work position by notched elastic tabs (47) projecting from a holding sleeve (34) connected to the tubular body (20) and the teeth (45) of which enter one of the grooves (43) arranged on the front part of the grooved shaft (40),
- connected to an arming sleeve (29) arranged behind the holding sleeve (34) which is mounted to slide in a tubular body (20) against the action of return means (30) pressing it against an internal flange (28) of this body, this connection being afforded by the entering of notched tabs (46) secured to this arming sleeve in one of the grooves (43) of the grooved shaft (40),
- and associated with movement means consisting of a longitudinal body (23a) which can be actuated by the second controls (25, 6c) and which, at the end of the travel for the distribution and placement of a fastener, pushes the arming sleeve (29) by the length of a catching bar (3) so as to move the grooved shaft (40), causing the tabs (47) of the holding sleeve (34) to part until their teeth enter the next groove, and causing the buffer rod (41) to advance by the magnitude of the catching bar (3) of a fastener.

5. Appliance for storing, distributing and placing fasteners according to Claim 4, **characterized in that** the longitudinal body (23a) causing the arming sleeve (29) to move consists of a tubular end-piece (23a) surrounding the grooved shaft (40) and projecting from the front end of a cage (23) secured to the plunger (42), the said cage containing the means (38) for returning the axial rod (39) of the slide (22) being secured to a control piston (25) entering the handgrip body (6) and collaborating with the second controls (6c).

6. Appliance for storing, distributing and placing fasteners according to Claim 1, **characterized in that** the rear end of the rod (39) connected to the magazine slide (22) rests against a lever (8) articulated in the handgrip body (6) and able to be moved by a two-finger fork (6a) of the handgrip (6) which is articulated in this body, while the piston (25) secured to the cage (23) and to the plunger (39) rests against a cam profile (6c) formed on the handgrip (6).

7. Appliance for storing, distributing and placing fasteners according to Claim 1, **characterized in that** the magazine slide (22) is formed of two plastic shells (22a, 22b) joined together along their median, longitudinal and vertical plane, the shell (22a) secured to the hollow and split needle (50) having two longitudinal slots (53, 52) arranged near its edges and able, one of them, to receive the catching bars (3) of the fasteners and the other the anchoring bars (2), the slot (53) for the catching bars (3) opening freely at the front end of the slide (22) while the slot (52) for the anchoring bars (2) opens onto an inclined plane (54) for guidance in the direction of the needle (50), while the other shell (22b) has a longitudinal channel (57, 58) coaxial with the needle (50) for the ejection plunger (42) and, in the accommodating housing (55) of the needle (A), a vertically inclined resting plane (56) for the connecting strip (4) of a fastener.

8. Appliance for storing, distributing and placing fasteners according to Claim 7, **characterized in that** the channel for the ejection plunger (42) is formed of a slot (57) made in the corresponding shell (22b) and of a slot (58) made in a plate (22c) attached inside the said shell (22b), the said plate (22c) having a width that allows it to be inserted between the slots (52, 53) made in the other shell (22a) for the anchoring bars (2) and the catching bars (3) respectively, and a thickness allowing it to delimit, with this other shell (22a), a passageway (60) for the passage of the connecting strips (3) of the fasteners.

9. Appliance for storing, distributing and placing fasteners according to Claim 1, **characterized in that** the elongate body (C) is made up of several plastic components assembled together and arranged in a tubular metal band (21).
